# EUROPEAN PATENT APPLICATION

(11) **EP 3 831 805 A1**
(43) Date of publication of application: **09.06.2021**
(21) Application number: 19214023.4
(22) Date of filing: 06.12.2019
(51) Int. Cl.: C07C 68/065, C07C 68/08, C07C 69/96, C07C 29/128, C07C 29/86, C07C 31/20

(54) **PROCESS FOR THE PREPARATION OF A DIALKYL CARBONATE AND AN ALKANEDIOL**

(71) Applicant: Shell Internationale Research Maatschappij B.V., 2596 HR The Hague (NL)
(72) Inventor: VAN DER HEIDE, Evert, 1031 HW Amsterdam (NL); FISCHER, Kai Jürgen, 1031 HW Amsterdam (NL)
(74) Representative: Shell Legal Services IP

(57) **Abstract**

The invention relates to a process for the preparation of a dialkyl carbonate and an alkanediol, comprising: (i) reacting an alkylene carbonate and an alkanol to obtain a product mixture containing unconverted alkylene carbonate, unconverted alkanol, dialkyl carbonate and alkanediol; (ii) contacting the product mixture obtained in step (i) with an extraction solvent and separating the extraction solvent phase comprising unconverted alkanol and alkanediol from the organic carbonate phase comprising unconverted alkylene carbonate and dialkyl carbonate, wherein the extraction solvent comprises an alkanol containing 2 or more hydroxyl groups; (iii) recovering the dialkyl carbonate; (iv) separating the extraction solvent phase obtained in step (ii) into a top stream comprising unconverted alkanol and a bottom stream comprising extraction solvent and alkanediol; and (v) recycling extraction solvent from the bottom stream obtained in step (iv) to step (ii). Further, the invention relates to a process for making a diaryl carbonate, comprising reacting an aryl alcohol with recovered dialkyl carbonate.

## Description

### Field of the invention

The present invention relates to a process for the preparation of a dialkyl carbonate and an alkanediol.

### Background of the invention

Dialkyl carbonates can be produced by reaction of alkylene carbonate with alkanol. Where alkylene carbonate (such as ethylene carbonate) is reacted with alkanol (such as ethanol), the products are dialkyl carbonate (such as diethyl carbonate) and alkanediol (such as monoethylene glycol). Such process is well-known and an example thereof is disclosed in US5359118. This document discloses a process in which di(C₁-C₄ alkyl) carbonates alkanediols are prepared by transesterification of an alkylene carbonate with a C₁-C₄ alkanol.

Typically, not all of the alkylene carbonate and alkanol is converted into dialkyl carbonate and an alkanediol, so that the resulting product mixture contains unconverted alkylene carbonate, unconverted alkanol, dialkyl carbonate and alkanediol. WO2008090108 discloses that such product mixture may be separated into a top stream containing unconverted alkanol and dialkyl carbonate and a bottom stream containing unconverted alkylene carbonate and alkanediol. Further, WO2008090108 discloses that said top stream may be separated into a top stream comprising unconverted alkanol that may be recycled to the reactor and a bottom stream containing dialkyl carbonate product. Further, it is disclosed in WO2008090108 that the above-mentioned bottom stream containing unconverted alkylene carbonate and alkanediol may be further separated by distillation. However, a disadvantage is that in such distillation an azeotrope of the alkanediol (e.g. ethylene glycol) and the unconverted alkylene carbonate (e.g. ethylene carbonate) may be formed, causing the resulting top stream to contain both alkanediol product and unconverted alkylene carbonate and the resulting bottom stream to contain unconverted alkylene carbonate which may be recycled to the reactor. WO2008090108 addresses this by a further purification procedure which involves first converting the alkylene carbonate in the latter top stream by hydrolysis, upon adding water, into alkanediol and carbon dioxide and further separating the resulting stream to obtain purified alkanediol. However, a disadvantage is that such additional purification procedure involving hydrolysis and further separation is cumbersome, and results in a lower amount of unconverted alkylene carbonate that is recycled to the reactor, thereby resulting in a lower overall yield of the desired dialkyl carbonate product.

A problem addressed in above-mentioned WO2008090108 is the potential formation of polyalkylene glycols (polyglycols), for example diethylene glycol, in the preparation of a dialkyl carbonate and an alkanediol from an alkylene carbonate and an alkanol. In the separation scheme as disclosed in said WO2008090108, such polyglycols end up in the above-mentioned bottom stream containing unconverted alkylene carbonate. By recycling the latter bottom stream to the reactor, these higher-boiling polyglycol by-products build up. In the invention of WO2008090108, such build-up is prevented by subjecting a part of such (bottom) stream containing alkylene carbonate and polyglycols to above-mentioned hydrolysis step, thereby obtaining alkylene glycol (alkanediol). However, a disadvantage is that only part of the unconverted alkylene carbonate may be recycled to the reactor and that the other part is converted to alkylene glycol, thereby resulting in a further reduced overall yield of the desired dialkyl carbonate product.

It is an object of the present invention to provide a simple, effective and efficient process for the preparation and recovery of a dialkyl carbonate and an alkanediol through reacting an alkylene carbonate with an alkanol, which process preferably does not have one or more of the above-mentioned disadvantages. Furthermore, such process preferably results in energy and/or capital savings.

### Summary of the invention

Surprisingly it was found that such process as described above may be provided by using an alkanol containing 2 or more hydroxyl groups as an extraction solvent in the separation of the product mixture containing unconverted alkylene carbonate, unconverted alkanol, dialkyl carbonate and alkanediol, said unconverted alkylene carbonate ending up in the organic carbonate phase and said alkanediol ending up in a separate extraction solvent phase.

Accordingly, the present invention relates to a process for the preparation of a dialkyl carbonate and an alkanediol, comprising:
(i) reacting an alkylene carbonate and an alkanol to obtain a product mixture containing unconverted alkylene carbonate, unconverted alkanol, dialkyl carbonate and alkanediol;
(ii) contacting the product mixture obtained in step (i) with an extraction solvent and separating the extraction solvent phase comprising unconverted alkanol and alkanediol from the organic carbonate phase comprising unconverted alkylene carbonate and dialkyl carbonate, wherein the extraction solvent comprises an alkanol containing 2 or more hydroxyl groups;
(iii) recovering the dialkyl carbonate;
(iv) separating the extraction solvent phase obtained in step (ii) into a top stream comprising unconverted alkanol and a bottom stream comprising extraction solvent and alkanediol; and
(v) recycling extraction solvent from the bottom stream obtained in step (iv) to step (ii).

An advantage of the present process for the preparation of a dialkyl carbonate and an alkanediol involving steps (i) to (v), is that the formation of an azeotrope of the alkanediol (e.g. ethylene glycol) and the unconverted alkylene carbonate (e.g. ethylene carbonate), as illustrated above with reference to WO2008090108, is avoided as in extraction step (ii) unconverted alkylene carbonate ends up in the organic carbonate phase and the alkanediol ends up in the extraction solvent phase. Thus, a cumbersome additional purification procedure involving hydrolysis and further separation as disclosed in said WO2008090108, is advantageously avoided in the present process wherein already in extraction step (ii) alkanediol and alkylene carbonate are separated from each other by means of extraction. As a consequence, there is no need to hydrolyse part of the unconverted alkylene carbonate as disclosed in said WO2008090108, so that more alkylene carbonate can be recycled to the reaction step resulting in a higher overall yield of the desired dialkyl carbonate product.

An additional advantage of the present process is that in case any polyalkylene glycols (polyglycols), for example diethylene glycol, are formed as a by-product, these end up in the bottom stream comprising extraction solvent and alkanediol obtained in step (iv) of the present process. Because in the present invention, any unconverted alkylene carbonate from said bottom stream does not need to be recycled to reaction step (i), there is advantageously neither any recycle of any polyglycols to reaction step (i). Therefore, in the present invention, there is no need to split a stream comprising unconverted alkylene carbonate and polyglycols, as in the process of above-mentioned WO2008090108 wherein one part is recycled to the reactor and the other part is hydrolysed. On the contrary, in the present invention, if polyglycols are formed they may be sent, in step (v), to extraction step (ii) together with extraction solvent from the bottom stream obtained in step (iv), in which extraction step (ii) such polyglycols containing 2 hydroxyl groups may advantageously function as additional extraction solvent. Furthermore, advantageously, by not having to split a stream comprising unconverted alkylene carbonate and polyglycols and then hydrolysing one part as taught in said WO2008090108, even more alkylene carbonate can be recycled to the reaction step resulting in a higher overall yield of the desired dialkyl carbonate product.

Further, the present invention relates to a process for making a diaryl carbonate, comprising reacting an aryl alcohol with dialkyl carbonate recovered in step (iii) of the above-described process.

### Brief description of the drawings

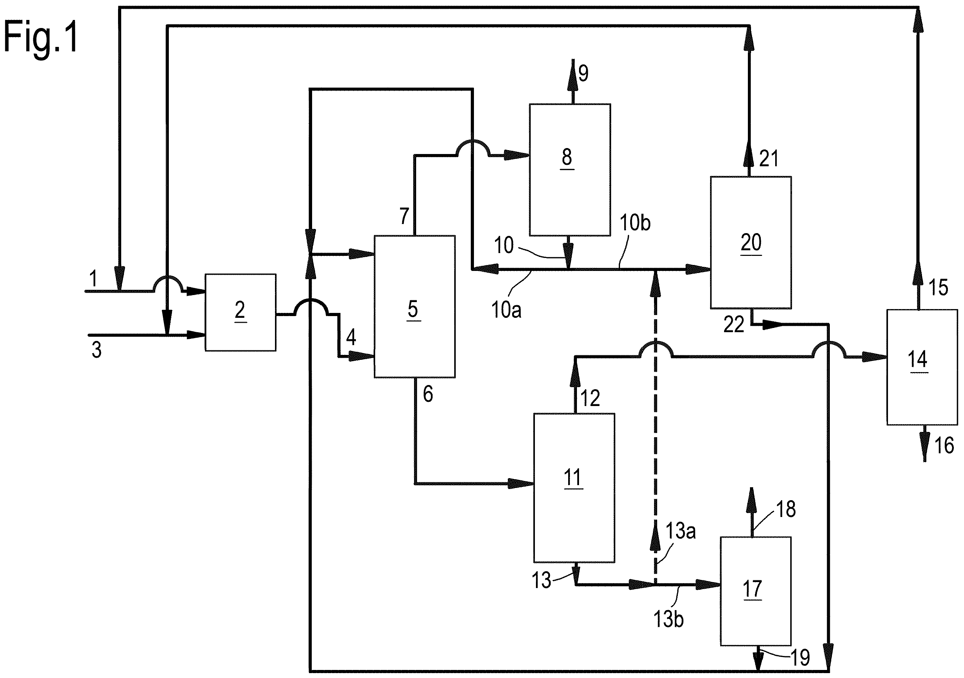

Figure 1 shows a process for the preparation of a dialkyl carbonate and an alkanediol in accordance with the present invention.

### Detailed description of the invention

In a case wherein in the present specification a process comprising two or more steps is disclosed, said process may comprise one or more intermediate steps between subsequent steps. Further, said process may comprise one or more additional steps preceding the first step and/or following the last step.

While in the present specification a process and mixtures or streams or catalysts used or produced in said process are described in terms of "comprising", "containing" or "including" one or more various described steps and components, respectively, they can also "consist essentially of" or "consist of" said one or more various described steps and components, respectively.

In the context of the present invention, in a case where a mixture or a stream or a catalyst comprises two or more components, these components are to be selected in an overall amount not to exceed 100%.

Further, where upper and lower limits are quoted for a property then a range of values defined by a combination of any of the upper limits with any of the lower limits is also implied.

Unless indicated otherwise, where in the present specification reference is made to a boiling point this means the boiling point at 760 mm Hg pressure.

The process of the present invention is characterized by contacting, in step (ii), the product mixture obtained in step (i) and containing unconverted alkylene carbonate, unconverted alkanol, dialkyl carbonate and alkanediol, with an extraction solvent which is an alkanol containing 2 or more hydroxyl groups, and separating the extraction solvent phase comprising unconverted alkanol and alkanediol from the organic carbonate phase comprising unconverted alkylene carbonate and dialkyl carbonate. Within the present specification, contacting the product mixture obtained in step (i) with an extraction solvent means that a stream comprising the product mixture obtained in step (i) is contacted with another stream comprising extraction solvent. In the process of the present invention, the extraction process in step (ii) is a process involving liquid-liquid extraction. Further, the organic carbonate phase (or raffinate phase) may be the upper phase and the extraction solvent phase (or extract phase) may be the lower phase.

In extraction step (ii), the extraction solvent comprises an alkanol containing 2 or more hydroxyl groups, suitably 3 or more hydroxyl groups, preferably of from 2 to 5 hydroxyl groups, more preferably of from 2 to 4 hydroxyl groups, more preferably of from 2 to 3 hydroxyl groups, most preferably 3 hydroxyl groups. Further, said alkanol may contain 2 to 9 carbon atoms, suitably 2 to 7 carbon atoms, more suitably 2 to 5 carbon atoms, most suitably 2 to 3 carbon atoms. Preferably, said alkanol contains at least 2 carbon atoms and at least 2 hydroxyl groups, in which alkanol each hydroxyl group is bonded to a different carbon atom. Suitable examples of said alkanol are monoethylene glycol, monopropylene glycol, glycerol, erythritol, xylitol and sorbitol, more suitably monoethylene glycol, monopropylene glycol and glycerol. More preferably, said alkanol is monoethylene glycol or glycerol, most preferably glycerol.

The product mixture obtained in step (i), to be treated in accordance with extraction step (ii) of the process of the present invention, also contains alkanediol containing 2 hydroxyl groups, such as monoethylene glycol. Such alkanediol may advantageously be used as additional extraction solvent. Thus, it is an advantage that alkanediol extraction solvent (such as monoethylene glycol) is a component that is already formed in the overall process, in reaction step (i), so that less extraction solvent needs to be added in extraction step (ii).

In extraction step (ii), any mixture of two or more of the above-described extraction solvents may be used. The weight ratio of said two or more extraction solvents may vary within a wide range. In the case of two extraction solvents, this weight ratio may be of from 0.1:10 to 10:1, suitably of from 0.5:1 to 5:1. The alkanediol containing 2 hydroxyl groups, such as monoethylene glycol, in the product mixture obtained in step (i), to be treated in accordance with extraction step (ii) of the process of the present invention, is considered as an extraction solvent and should therefore be included in the above-mentioned weight ratio of said two or more extraction solvents. A preferred mixture of extraction solvents is a mixture of an alkanol containing 2 hydroxyl groups, for example an alkanediol such as monoethylene glycol, with an alkanol containing 3 hydroxyl groups, for example glycerol. An advantage of using such alkanediol is that,

Preferably, in extraction step (ii), the density of the lower phase is at least 0.05 g/ml, more preferably at least 0.10 g/ml, even more preferably at least 0.15 g/ml, most preferably at least 0.20 g/ml higher than the density of the upper phase. Where needed, such density difference may also be achieved by adding additives that change the density of one of the phases, such additives including salts.

Advantageously, in the present process, the contacting, in step (ii), of the product mixture obtained in step (i) and containing unconverted alkylene carbonate, unconverted alkanol, dialkyl carbonate and alkanediol, with an extraction solvent which is an alkanol containing 2 or more hydroxyl groups, results in the formation of an extraction solvent phase comprising unconverted alkanol and alkanediol and an organic carbonate phase comprising unconverted alkylene carbonate and dialkyl carbonate, thereby separating unconverted alkylene carbonate from alkanediol.

The time for contacting, in step (ii), of the product mixture obtained in step (i) with the extraction solvent in the present process should be sufficient to complete the extraction process. Said contacting time may be in the order of 10 seconds to 5 hours, for example 10 seconds to 2 hours.

The temperature at which extraction step (ii) is performed, that is to say the temperature of the biphasic mixture, may be comprised in the range of from 0 to 150 °C, for example 10 to 100 °C. Advantageously, since the feed to extraction step (ii) is a product mixture obtained by reacting an alkylene carbonate and an alkanol and containing unconverted alkylene carbonate, unconverted alkanol, dialkyl carbonate and alkanediol, the feed does not have to be cooled before sending it to extraction step (ii).

A suitable weight ratio of extraction solvent to the product mixture obtained in step (i), is comprised in the range of from 10:1 to 1:10, for example 5:1 to 1:5. The alkanediol containing 2 hydroxyl groups, such as monoethylene glycol, in the product mixture obtained in step (i), to be treated in accordance with extraction step (ii) of the process of the present invention, is considered as an extraction solvent and should therefore only be included as "extraction solvent" in the above-mentioned weight ratio of extraction solvent to the product mixture obtained in step (i).

The pressure at which extraction step (ii) is carried out, may be subatmospheric, atmospheric or superatmospheric pressure, preferably atmospheric or superatmospheric pressure.

The total amount of unconverted alkanol in the product mixture that is subjected to above-mentioned extraction step (ii) may be comprised in the range of from 10 parts per million by weight (ppmw) to 10 wt.%, specifically 100 ppmw to 9 wt.%, more specifically 0.1 to 8 wt.%, more specifically 0.3 to 7 wt.%, more specifically 0.5 to 6 wt.% and most specifically 0.5 to 5 wt.%.

It may be preferred, for example in a case where there is a relatively large amount of unconverted alkanol in the product mixture obtained in step (i), to first remove unconverted alkanol from that product mixture before subjecting it to above-mentioned extraction step (ii). Preferably 10 to 80%, more preferably 20 to 70%, of unconverted alkanol may be removed in such case from the product mixture obtained in step (i). Such removal of unconverted alkanol may be effected in any known way, for example by distillation including a vapour-liquid flash.

Further, in extraction step (ii) of the present process, the extraction solvent phase should be separated from the organic carbonate phase. Any skilled person can find a suitable method of separating the extraction solvent phase from the organic carbonate phase in extraction step (ii) of the present process.

Step (iii) of the present process for the preparation of a dialkyl carbonate and an alkanediol comprises recovering the dialkyl carbonate. Said step (iii) may be effected by means of distillation. The dialkyl carbonate may be recovered by separating the organic carbonate phase comprising unconverted alkylene carbonate and dialkyl carbonate obtained in step (ii) to obtain a top stream containing dialkyl carbonate and a bottom stream containing unconverted alkylene carbonate. Unconverted alkylene carbonate in said bottom stream may be recycled to step (i). In a case wherein the organic carbonate phase obtained in step (ii) additionally comprises alkanediol and/or extraction solvent, said bottom stream additionally comprises alkanediol and/or extraction solvent. In the latter case, it is preferred that said bottom stream comprises less than 15 wt.% of alkanediol.

In a case wherein the organic carbonate phase obtained in step (ii) additionally comprises alkanediol and/or extraction solvent as mentioned above and/or in a case wherein the above-mentioned bottom stream containing unconverted alkylene carbonate is combined with alkanediol and extraction solvent from a stream originating from the extraction solvent phase obtained in step (ii), said bottom stream or the combined stream, which comprises unconverted alkylene carbonate and additionally alkanediol and/or extraction solvent, may be separated into a top stream containing unconverted alkylene carbonate and a bottom stream containing alkanediol and/or extraction solvent. Unconverted alkylene carbonate in said top stream may be recycled to step (i). Alkanediol and/or extraction solvent in said bottom stream may be recycled to step (ii). In a case where step (iii) results in a bottom stream containing unconverted alkylene carbonate and additionally alkanediol and/or extraction solvent, said bottom stream may be split into two streams. One split stream may then be recycled to step (ii). As described above, the other split stream may be separated into a top stream containing unconverted alkylene carbonate and a bottom stream containing alkanediol and/or extraction solvent, optionally after first combining said other split stream with a split stream resulting from step (v) and comprising alkanediol and extraction solvent as described below. Said separations may be effected by means of distillation.

Step (iv) of the present process for the preparation of a dialkyl carbonate and an alkanediol comprises separating the extraction solvent phase obtained in step (ii) into a top stream comprising unconverted alkanol, which may be recycled to step (i), and a bottom stream comprising extraction solvent and alkanediol. Said separation may be effected by means of distillation. In a case where the extraction solvent phase additionally comprises unconverted alkylene carbonate, said bottom stream additionally comprises unconverted alkylene carbonate. In the latter case, it is preferred that said bottom stream comprises less than 50 wt.% of unconverted alkylene carbonate.

Step (v) of the present process for the preparation of a dialkyl carbonate and an alkanediol comprises recycling extraction solvent from the bottom stream obtained in step (iv), comprising extraction solvent and alkanediol, to step (ii). Step (v) may comprise separating the bottom stream obtained in step (iv) into a top stream comprising alkanediol, thereby recovering alkanediol as one of the products, and a bottom stream comprising extraction solvent which may be recycled to step (ii). Alternatively, for example in the above-mentioned case wherein the organic carbonate phase obtained in step (ii) additionally comprises alkanediol and/or extraction solvent and/or in a case wherein the bottom stream obtained in step (iv) additionally comprises unconverted alkylene carbonate, the bottom stream obtained in step (iv) may be split into two streams. As described above, one split stream may then be separated into a top stream comprising alkanediol, thereby recovering alkanediol as one of the products, and a bottom stream comprising extraction solvent which may be recycled to step (ii). The other split stream may be combined with a bottom stream obtained in step (iii) comprising unconverted alkylene carbonate and additionally alkanediol and/or extraction solvent, and then separated in a way as described above. Said separations may be effected by means of distillation.

In the present process, when recycling extraction solvent, such as extraction solvent from the bottom stream obtained in step (iv), to step (ii), a portion of the extraction solvent containing recycle stream(s) may be removed as a bleed to prevent a build-up of any heavies. In case of such bleed, an extraction solvent containing make-up stream needs to be fed to step (ii).

The separations and recovery of products in steps (iii), (iv) and (v) may be effected in any known way.

Step (i) of the present process for the preparation of a dialkyl carbonate and an alkanediol comprises reacting an alkylene carbonate and an alkanol to obtain a product mixture containing unconverted alkylene carbonate, unconverted alkanol, dialkyl carbonate and alkanediol.

Thus, step (i) includes the reaction of an alkylene carbonate with an alkanol. Said alkylene carbonate may be a C₂-C₆ alkylene carbonate, more suitably a C₂-C₄ alkylene carbonate, most suitably a C₂-C₃ alkylene carbonate. Preferably, said alkylene carbonate is ethylene carbonate or propylene carbonate, most preferably ethylene carbonate. The nature of the alkylene carbonate determines the nature of the alkanediol product: for example, reaction of ethylene carbonate with an alkanol results in monoethylene glycol, which is 1,2-ethanediol (the alkanediol).

Further, said alkanol may be a C₁-C₄ alkanol, more suitably a C₁-C₃ alkanol, most suitably a C₂-C₃ alkanol. Preferably, said alkanol contains 1 or 2 hydroxy groups, most preferably 1 hydroxy group. Further, preferably, said alkanol is methanol, ethanol or isopropanol, more preferably ethanol or isopropanol, most preferably ethanol. The nature of the alkanol determines the nature of the dialkyl carbonate product: for example, reaction of an alkylene carbonate with ethanol results in diethyl carbonate (the dialkyl carbonate).

Preferably, in step (i) for the preparation of a dialkyl carbonate and an alkanediol a catalyst is used, more specificly a transesterification catalyst.

The transesterification catalyst that may be used in step (i) may be one of many suitable homogeneous and heterogeneous transesterification catalysts known from prior art.

For example, suitable homogeneous transesterification catalysts have been described in US5359118 and include hydrides, oxides, hydroxides, alkanolates, amides, or salts of alkali metals, i.e., lithium, sodium, potassium, rubidium and cesium. Preferred homogeneous transesterification catalysts are hydroxides or alkanolates of potassium or sodium. Other suitable homogeneous transesterification catalysts are alkali metal salts, such as acetates, propionates, butyrates, or carbonates. Suitable catalysts are described in US5359118 and the references mentioned therein, such as EP274953A, US3803201, EP1082A, and EP180387A.

As mentioned above, it is also possible to employ a heterogeneous transesterification catalyst. In the present process, the use of a heterogeneous transesterification catalyst in step (i) is preferred. Suitable heterogeneous catalysts include ion exchange resins that contain functional groups. Suitable functional groups include tertiary amine groups and quaternary ammonium groups, and also sulphonic acid and carboxylic acid groups. Further suitable catalysts include alkali metal and alkaline earth metal silicates. Suitable catalysts have been disclosed in US4062884 and US4691041. The heterogeneous catalyst may be selected from ion exchange resins comprising a polystyrene matrix and tertiary amine functional groups. An example is Amberlyst A-21 (ex Rohm & Haas) comprising a polystyrene matrix to which N,N-dimethylamine groups have been attached. Eight classes of transesterification catalysts, including ion exchange resins with tertiary amine and quaternary ammonium groups, are disclosed in J F Knifton et al., J. Mol. Catal, 67 (1991) 389ff.

A suitable heterogeneous transesterification catalyst may be a catalyst comprising an element from Group 4 (such as titanium), Group 5 (such as vanadium), Group 6 (such as chromium or molybdenum) or Group 12 (such as zinc) of the Periodic Table of the Elements, or tin or lead, or a combination of such elements, such as a combination of zinc with chromium (for example zinc chromite). Said elements may be present in the catalyst as an oxide, such as zinc oxide. Preferably, in step (i) a heterogeneous catalyst comprising zinc is used.

The conditions in step (i) include a temperature of from 10 to 200 °C, and a pressure of from 0.5 to 50 bara (5x10⁴ to 5x10⁶ N/m²). Preferably, especially in co-current operation, said pressure ranges from 1 to 20 bar, more preferably 1.5 to 20 bar, most preferably 2 to 15 bar, and said temperature ranges from 30 to 200 °C, more preferably 40 to 170 °C, most preferably 50 to 150 °C.

Further, preferably an excess of the alkanol over the alkylene carbonate is used in step (i). The molar ratio of alkanol to alkylene carbonate in step (i) is suitably of from 1.01:1 to 25:1, preferably of from 2:1 to 20:1, more preferably of from 3:1 to 15:1, most preferably from 3:1 to 13:1.

Still further, the weight hourly space velocity (WHSV) in step (i) may suitably range of from 0.1 to 100 kg/kg_{cat}.hr ("kg_{cat}" refers to the catalyst amount), more suitably 0.5 to 50 kg/kg_{cat}.hr, more suitably 1 to 20 kg/kg_{cat}.hr, more suitably 1 to 10 kg/kg_{cat}.hr.

Step (i) may be carried out in a reactive distillation column, as described in US5359118. This would entail that the reaction is carried out counter-currently. The distillation column may contain trays with bubble caps, sieve trays, or Raschig rings. The skilled person will realise that several types of packings of catalyst and several tray configurations will be possible. Suitable columns have been described in, e.g., Ullmann's Encyclopedia of Industrial Chemistry, 5th ed. Vol. B4, pp 321 ff, 1992.

The alkylene carbonate will generally have a higher boiling point than the alkanol. In the case of ethylene and propylene carbonate the atmospheric boiling points are above 240 °C. Therefore, in general, the alkylene carbonate will be fed at the upper part of a reactive distillation column and alkanol will be fed at the lower part of such column. The alkylene carbonate will flow downwardly, and the alkanol will flow upwardly.

Preferably, step (i) is conducted in a co-current manner. A suitable way to operate is to carry out the reaction in a trickle-flow manner wherein the reactants part in vapour phase and part in liquid phase drip down over the catalyst. A more preferred way to operate step (i) is in a reactor with only liquids. A suitable reaction zone of this type is a pipe-type reaction zone wherein the reaction is conducted in a plug flow manner. For example, step (i) may be carried out in one plug flow reactor or in a series of two or more plug flow reactors. This will enable the reaction to approach equilibrium.

A further possibility is to conduct step (i) in a continuously stirred tank reactor (CSTR). In the latter case the effluent from the CSTR is preferably subjected to a post-reaction in a plug flow reactor so that the reaction can approach equilibrium.

Step (i) is preferably carried out continuously. Further, unconverted alkylene carbonate and alkanol are preferably recycled.

In addition to unconverted alkanol and unconverted alkylene carbonate and the desired dialkyl carbonate and alkanediol products, the product mixture obtained in step (i) of the present process may also contain an ether alkanol impurity.

At various points within a total process for producing dialkyl carbonate from alkylene oxide via alkylene carbonate, organic carbonate streams containing one or more ether alkanol (i.e. alkoxy alkanol) impurities may be produced. For example, in a reactor where ethanol and ethylene carbonate are reacted into diethyl carbonate and monoethylene glycol, a side-reaction of ethanol with ethylene oxide, formed by back-reaction of ethylene carbonate into ethylene oxide and carbon dioxide, into 2-ethoxyethanol (ethyl oxitol or ethylene glycol monoethyl ether) may take place. Further, ethyl oxitol may be formed by a side-reaction of ethanol with ethylene carbonate in such a way that carbon dioxide is released and ethyl oxitol is produced. Still further, a side-reaction between ethanol and monoethylene glycol may take place producing ethyl oxitol and water. Still even further, ethyl oxitol may be formed via decarboxylation of hydroxyethyl ethyl carbonate.

Therefore, the product stream from a reactor where ethanol and ethylene carbonate are reacted into diethyl carbonate and monoethylene glycol, may comprise unconverted ethanol, unconverted ethylene carbonate, diethyl carbonate, monoethylene glycol and the above-mentioned ethyl oxitol impurity. The presence of said alkoxy alkanol impurity may be detrimental in any subsequent production process. Said alkoxy alkanol impurity may for example end up in the dialkyl carbonate that is used as a starting material for the synthesis of diphenyl carbonate from said dialkyl carbonate and phenol. For example, in a case where the dialkyl carbonate is diethyl carbonate and the alkoxy alkanol impurity is ethyl oxitol, said ethyl oxitol may react with the phenol starting material and/or with the diphenyl carbonate product.

Direct reaction of phenol and ethyl oxitol may result in the production of phenyl 2-ethoxyethyl ether, and hence in the loss of valuable phenol reactant. Further, such reaction results in the introduction of undesired chemicals in the process and therefore to separation issues.

Reaction of diphenyl carbonate with ethyl oxitol results in product loss as phenyl 2-ethoxyethyl carbonate is produced. Further, the latter product acts as a "poison" in any subsequent polymerisation of diphenyl carbonate into polycarbonate material. For example, when diphenyl carbonate is reacted with bis-phenol A (BPA), polycarbonate and phenol are formed. Diphenyl carbonate can react with BPA since phenol is a relatively good leaving group. Dialkyl carbonates (such as diethyl carbonate) however cannot be used to produce polycarbonate by reaction with BPA, since alkanols are not good leaving groups. Alkoxy alkanols (such as ethyl oxitol) are neither good leaving groups. Therefore, in case phenyl 2-ethoxyethyl carbonate is present in a diphenyl carbonate feed to be reacted with BPA, phenol will be released easily from said phenyl 2-ethoxyethyl carbonate but not ethyl oxitol which will consequently stop the polymerization process at one end of the chain. Consequently, phenyl 2-ethoxyethyl carbonate has to be removed from diphenyl carbonate before the latter is contacted with BPA.

The above exemplifies that in a case where an organic carbonate stream containing an ether alkanol impurity is formed, it is desired to remove said ether alkanol impurity before any subsequent process takes place wherein the organic carbonate is transformed into a valuable end product. For example, it is needed to remove any ethyl oxitol impurity from a diethyl carbonate stream containing said impurity before reaction of the diethyl carbonate with phenol takes place.

Referring to the above example where ethanol and ethylene carbonate have been reacted into diethyl carbonate and monoethylene glycol, the product stream also containing unconverted ethanol and ethylene carbonate and ethyl oxitol side-product, may be separated by means of distillation. The boiling points for the various components in said product stream are mentioned in the table below.

| | Boiling point (°C) |
|---|---|
| ethanol | 78.4 |
| diethyl carbonate | 126-128 |
| ethyl oxitol | 135 |
| monoethylene glycol | 197.3 |
| ethylene carbonate | 260.4 |

The distillation as referred to above may result in a top stream containing diethyl carbonate and unconverted ethanol and a bottom stream containing monoethylene glycol and unconverted ethylene carbonate. Most likely, all of the ethyl oxitol ends up in the top stream. However, depending on the specific conditions under which distillation is carried out, part of the ethyl oxitol may end up in the bottom stream. Subsequently, said top stream may be further separated by means of distillation into a top stream containing unconverted ethanol which can be recycled to the reactor where diethyl carbonate and monoethylene glycol are produced, and a bottom stream containing diethyl carbonate and the ethyl oxitol impurity.

As discussed above, before an organic carbonate is transformed into a valuable end product in any subsequent process, the ether alkanol impurity has to be removed therefrom as that might interfere said subsequent process and/or any further processes. For the above example, this means that the ethyl oxitol impurity should be removed from the bottom stream containing diethyl carbonate and the ethyl oxitol impurity. In principle, ethyl oxitol and diethyl carbonate could be separated by means of a further distillation step. However because of the small difference in boiling point between diethyl carbonate and ethyl oxitol (see above table), such separation is very cumbersome requiring many distillation steps and stages.

Similarly, in a case wherein ethanol and propylene carbonate have been reacted into diethyl carbonate and monopropylene glycol, ethyl proxitol may be formed as an ether alkanol impurity. Ethyl proxitol (propylene glycol monoethyl ether) comprises 1-ethoxy-2-propanol and/or 2-ethoxy-1-propanol. In this case there is also a small difference in boiling point between diethyl carbonate (126-128 °C) and the ether alkanol impurity (1-ethoxy-2-propanol: 132 °C; 2-ethoxy-1-propanol: 138 °C), making separation by distillation very cumbersome.

An additional advantage of the present process for the preparation of a dialkyl carbonate and an alkanediol involving steps (i) to (v), in a case wherein the product mixture obtained in step (i) also contains an ether alkanol impurity as described above, is that extraction step (ii) of the present process results in the formation of an extraction solvent phase comprising unconverted alkanol, alkanediol and the ether alkanol impurity and an organic carbonate phase comprising unconverted alkylene carbonate and dialkyl carbonate, thereby not only separating unconverted alkylene carbonate from alkanediol but also separating the ether alkanol impurity from the organic carbonates, which ether alkanol impurity might have interfered in any subsequent process using said organic carbonates, especially dialkyl carbonate, if the ether alkanol impurity would not have been removed.

Thus, in a case wherein the product mixture obtained in step (i) also contains an ether alkanol impurity as described above, extraction step (ii) comprises contacting the product mixture obtained in step (i) with an extraction solvent as described above and separating the extraction solvent phase comprising unconverted alkanol, alkanediol and the ether alkanol impurity from the organic carbonate phase comprising unconverted alkylene carbonate and dialkyl carbonate.

In extraction step (ii) of the process of the present invention, an ether alkanol impurity may advantageously be removed. An ether alkanol is identical to an alkoxy alkanol, and both terms are used interchangeably in the present specification. An alkoxy alkanol is an alkanol of formula R₁OH wherein R₁ is an alkoxyalkyl group. In a case wherein the product mixture obtained in step (i) also contains an ether alkanol impurity as described above, the alkoxy part in said alkoxyalkyl group is directly or indirectly derived from the alkanol used in step (i), whereas the alkyl part in said alkoxyalkyl group is directly or indirectly derived from the alkylene carbonate used in step (i). Therefore, the same preferences apply as discussed above with reference to the alkanol and alkylene carbonate used in step (i). For example, the alkoxy part in said alkoxyalkyl group is preferably methoxy, ethoxy or isopropoxy, more preferably ethoxy or isopropoxy, most preferably ethoxy. Further, for example, the alkyl part in said alkoxyalkyl group is preferably ethyl or propyl, most preferably ethyl. The ether alkanol impurity may for example be 2-ethoxyethanol or 1-ethoxy-2-propanol and/or 2-ethoxy-1-propanol.

In a particularly preferred embodiment of the present process for the preparation of a dialkyl carbonate and an alkanediol, the alkylene carbonate is ethylene carbonate, the alkanol is ethanol, the dialkyl carbonate is diethyl carbonate, the alkanediol is monoethylene glycol and the optional ether alkanol impurity is 2-ethoxyethanol. 2-Ethoxyethanol may also be referred to as ethyl oxitol.

In another particularly preferred embodiment of the present process for the preparation of a dialkyl carbonate and an alkanediol, the alkylene carbonate is propylene carbonate, the alkanol is ethanol, the dialkyl carbonate is diethyl carbonate, the alkanediol is monopropylene glycol and the optional ether alkanol impurity is ethyl proxitol (propylene glycol monoethyl ether) which comprises 1-ethoxy-2-propanol and/or 2-ethoxy-1-propanol.

In a case wherein the product mixture obtained in step (i) also contains an ether alkanol impurity as described above, the total amount of unconverted alkanol and the ether alkanol impurity in the product mixture that is subjected to above-mentioned extraction step (ii) may be comprised in the range of from 10 parts per million by weight (ppmw) to 10 wt.%, specifically 100 ppmw to 9 wt.%, more specifically 0.1 to 8 wt.%, more specifically 0.3 to 7 wt.%, more specifically 0.5 to 6 wt.% and most specifically 0.5 to 5 wt.%.

In a case wherein the product mixture obtained in step (i) also contains an ether alkanol impurity as described above, step (iv) of the present process comprises separating the extraction solvent phase obtained in step (ii) into a top stream comprising unconverted alkanol and the ether alkanol impurity and a bottom stream comprising extraction solvent and alkanediol. Said top stream comprising unconverted alkanol and the ether alkanol impurity may be separated into a top stream comprising unconverted alkanol, which may be recycled to step (i), and a bottom stream comprising the ether alkanol impurity. Said separation may be effected by means of distillation. In this way, at least a portion of any ether alkanol impurity may advantageously be recovered as a high purity ether alkanol product.

Further, the present invention relates to a process for making a diaryl carbonate, comprising reacting an aryl alcohol with dialkyl carbonate recovered in step (iii) of the above-described process.

Thus, the present invention relates to a process for making a diaryl carbonate, comprising contacting, in the presence of a transesterification catalyst, an aryl alcohol with dialkyl carbonate recovered in step (iii) of the above-described process.

Further, the present invention also relates to a process for making a diaryl carbonate, comprising the preparation of a dialkyl carbonate and an alkanediol in accordance with the above-described process, and then contacting, in the presence of a transesterification catalyst, an aryl alcohol with dialkyl carbonate recovered in step (iii) of the above-described process.

Preferably, in the above-described processes for making a diaryl carbonate, the diaryl carbonate is diphenyl carbonate and the aryl alcohol is phenol.

In addition, the above-described transesterification catalyst and other transesterification conditions, as described for step (i), are equally applicable to said processes for making a diaryl carbonate.

The present process for the preparation of a dialkyl carbonate and an alkanediol involving steps (i) to (v), including extraction step (ii) as one of the steps, is illustrated in Figure 1.

In Figure 1, ethanol is passed via line 1 into a reactor 2. Reactor 2 can suitably be a continuously stirred tank reactor. Via line 3 ethylene carbonate is also fed into reactor 2. A transesterification catalyst may be present or may be fed continuously to the reactor. The catalyst may be mixed with one of the reactants or fed to the reactor via a separate line (not shown). A product mixture containing unconverted ethylene carbonate, unconverted ethanol, diethyl carbonate, ethylene glycol (an alkanediol) and 2-ethoxyethanol (an ether alkanol impurity; ethyl oxitol) is withdrawn from reactor 2 via line 4. Via line 4 the mixture is passed to an extraction column 5 at a lower part thereof. In said column, said product mixture is subjected to liquid-liquid extraction using glycerol as extraction solvent which is fed to the column at an upper part thereof.

The resulting top stream from extraction column 5 in line 7 comprises diethyl carbonate, unconverted ethylene carbonate, ethylene glycol and glycerol, and is subjected to distillation in a distillation column 8, resulting in a top stream comprising diethyl carbonate that is recovered via line 9 and a bottom stream comprising unconverted ethylene carbonate, ethylene glycol and glycerol in line 10. Stream 10 is split into two substreams 10a and 10b. Substream 10a is recycled to extraction column 5.

The resulting bottom stream from extraction column 5 in line 6 comprises unconverted ethanol, ethyl oxitol, ethylene glycol and glycerol. Optionally, said bottom stream may be split (not shown in Figure 1, but shown in Figure 1 in relation to stream 15), wherein one substream is recycled to extraction column 5 at an upper part thereof and the other substream is sent to a distillation column 11. Stream 6 (without split) is subjected to distillation in distillation column 11, resulting in a top stream comprising unconverted ethanol and ethyl oxitol in line 12 and a bottom stream comprising ethylene glycol and glycerol in line 13. The stream in line 12 is subjected to distillation in a distillation column 14, resulting in a top stream comprising unconverted ethanol that is recycled via lines 15 and 1 to reactor 2, and a bottom stream comprising ethyl oxitol in line 16. The stream in line 13 is optionally split into two substreams 13a and 13b. Substream 13b (with split) or stream 13 (without split) is subjected to distillation in a distillation column 17, resulting in a top stream comprising ethylene glycol that is recovered via line 18 and a bottom stream comprising glycerol that is sent via line 19 to extraction column 5.

Substream 10b comprising unconverted ethylene carbonate, ethylene glycol and glycerol, optionally in combination with substream 13a, is subjected to distillation in a distillation column 20, resulting in a top stream comprising unconverted ethylene carbonate that is recycled via lines 21 and 3 to reactor 2, and a bottom stream comprising ethylene glycol and glycerol that is sent via line 22 to extraction column 5.

Extraction step (ii) of the process of the present invention is further illustrated by the following Examples.

### Examples

Liquid-Liquid Equilibrium (LLE) data measurements were performed in a stirred and thermoregulated glass cell. After equilibration and phase settling samples were taken from both phases and analyzed by gas chromatography. The molar concentrations of each component in each of the two phases were determined.

For the quinary system 2-ethoxyethanol (oxitol or EE) + diethyl carbonate (DEC) + ethylene carbonate (EC) + monoethylene glycol (MEG) + glycerol (GLY), LLE data were measured at ambient pressure and at a temperature of 50 °C with a 2-ethoxyethanol feed concentration that was varied (1, 3, 5 and 7.5 wt.%). The weight ratio of DEC:EC:MEG:GLY in the feed was kept constant at 1:1:1:1.

Further, for the quinary system ethanol (EtOH) + DEC + EC + MEG + GLY, LLE data were also measured in the same way, except that the ethanol feed concentrations were 1, 3, 5 and 6 wt.%, respectively.

For each component in each of the above two quinary systems, the mole fraction in each of the two phases was determined. The mole fraction of a component in the upper phase (raffinate phase) is referred to in Tables 1 and 2 below as x(component), for example x(DEC). The mole fraction of a component in the lower phase (extract phase) is referred to in Tables 1 and 2 below as y(component), for example y(DEC). These mole fraction data can be converted into distribution coefficients (K-values), where K(component) is equal to the upper phase mole fraction divided by the lower phase mole fraction for a certain component, for example K(DEC). The relative selectivity alpha(component/oxitol or EtOH) can be defined as the ratio of the K-values of DEC and oxitol (EE) or ethanol (EtOH), for example K(DEC)/K(EE) in the oxitol containing quinary system and K(DEC)/K(EtOH) in the ethanol containing quinary system. When this relative selectivity is 1, there is no difference in selectivity between a component and oxitol or EtOH with respect to the two phases. The efficiency of a solvent to enhance the separation of oxitol and/or EtOH from an organic carbonate, such DEC and EC, can be assessed by monitoring the change of the relative selectivity alpha when adding such solvent.

Table 1 below shows for the oxitol containing quinary system the LLE data including the derived K-values and relative selectivities (as compared with oxitol). Table 1 below shows for the ethanol containing quinary system the LLE data including the derived K-values and relative selectivities (as compared with ethanol). An alpha value (relative selectivity) higher than 1 means that the first component will end up in the upper (raffinate) phase and the second component (oxitol or EtOH) will end up in the lower (extract) phase in a liquid-liquid extraction column.

The data in Tables 1 and 2 show firstly that by adding monoethylene glycol and glycerol the relative selectivity of organic carbonates (such as DEC and EE) versus oxitol or EtOH is higher than 1 in the full range of compositions evaluated (with varying oxitol or EtOH concentrations in the feed), and that therefore there is a difference in selectivity between the organic carbonates and oxitol or EtOH with respect to the two phases, and secondly that the organic carbonates will move up in a liquid-liquid extraction column, and that oxitol or EtOH will move down together with the monoethylene glycol and glycerol. Thus, advantageously, monoethylene glycol and glycerol are good extraction solvents for separating oxitol and/or EtOH from organic carbonates (such as DEC and EE) in a liquid-liquid extraction process. Surprisingly, monoethylene glycol and glycerol are solvents that preferably dissolve oxitol and/or EtOH and to a lesser extent organic carbonates, such as DEC and ethylene carbonate. In a further step, the oxitol and/or EtOH may easily be separated from monoethylene glycol and glycerol (e.g. by distillation) after which the monoethylene glycol and glycerol can advantageously be reused as extraction solvent in liquid-liquid extraction of mixtures containing organic carbonate and oxitol and/or EtOH.

**Table 1: Liquid-liquid extraction data for 2-ethoxyethanol (EE)**

| EE (wt.%) | x(EE) | x(DEC) | x(EC) | x (MEG) | x(GLY) |
|---|---|---|---|---|---|
| 1 | 0.0106 | 0.4850 | 0.3174 | 0.1364 | 0.0507 |
| 3 | 0.0307 | 0.4480 | 0.2980 | 0.1590 | 0.0643 |
| 5 | 0.0569 | 0.4231 | 0.2686 | 0.1866 | 0.0648 |
| 7.5 | 0.0763 | 0.3650 | 0.2345 | 0.2261 | 0.0980 |

| | y(EE) | y(DEC) | y(EC) | y(MEG) | y(GLY) |
|---|---|---|---|---|---|
| 1 | 0.0102 | 0.0594 | 0.1018 | 0.5148 | 0.3138 |
| 3 | 0.0259 | 0.0622 | 0.0881 | 0.5301 | 0.2938 |
| 5 | 0.0471 | 0.0768 | 0.0889 | 0.5020 | 0.2852 |
| 7.5 | 0.0611 | 0.0972 | 0.0987 | 0.4475 | 0.2955 |

| | K(EE) | K(DEC) | K(EC) | K(MEG) | K(GLY) |
|---|---|---|---|---|---|
| 1 | 1.039 | 8.165 | 3.118 | 0.265 | 0.162 |
| 3 | 1.185 | 7.203 | 3.383 | 0.300 | 0.219 |
| 5 | 1.208 | 5.509 | 3.021 | 0.372 | 0.227 |
| 7.5 | 1.249 | 3.755 | 2.376 | 0.505 | 0.332 |

| | K(EE)/K(EE) | K(DEC)/K(EE) | K(EC)/K(EE) | K(MEG)/K(EE) | K(GLY)/K(EE) |
|---|---|---|---|---|---|
| 1 | 1 | 7.857 | 3.000 | 0.255 | 0.155 |
| 3 | 1 | 6.076 | 2.854 | 0.253 | 0.185 |
| 5 | 1 | 4.560 | 2.501 | 0.308 | 0.188 |
| 7.5 | 1 | 3.007 | 1.903 | 0.405 | 0.266 |

**Table 2: Liquid-liquid extraction data for ethanol (EtOH)**

| EtOH (wt.%) | x(EtOH) | x(DEC) | x(EC) | x(MEG) | x(GLY) |
|---|---|---|---|---|---|
| 1 | 0.0231 | 0.4872 | 0.2552 | 0.1548 | 0.0798 |
| 3 | 0.0544 | 0.4396 | 0.2538 | 0.1794 | 0.0727 |
| 5 | 0.0932 | 0.3825 | 0.2240 | 0.2272 | 0.0731 |
| 6 | 0.1087 | 0.3622 | 0.2241 | 0.2145 | 0.0905 |

| | y(EtOH) | y(DEC) | y(EC) | y(MEG) | y(GLY) |
|---|---|---|---|---|---|
| 1 | 0.0273 | 0.0650 | 0.0925 | 0.5440 | 0.2712 |
| 3 | 0.0579 | 0.0807 | 0.0901 | 0.4906 | 0.2808 |
| 5 | 0.0984 | 0.1003 | 0.0965 | 0.4598 | 0.2449 |
| 6 | 0.1121 | 0.1232 | 0.1051 | 0.4305 | 0.2291 |

| | K(EtOH) | K(DEC) | K(EC) | K(MEG) | K(GLY) |
|---|---|---|---|---|---|
| 1 | 0.846 | 7.495 | 2.759 | 0.285 | 0.294 |
| 3 | 0.940 | 5.447 | 2.817 | 0.366 | 0.259 |
| 5 | 0.947 | 3.814 | 2.321 | 0.494 | 0.298 |
| 6 | 0.970 | 2.940 | 2.132 | 0.498 | 0.395 |

| | K(EtOH)/K(EtOH) | K(DEC)/K(EtOH) | K(EC)/K(EtOH) | K(MEG)/K(EtOH) | K(GLY)/K(EtOH) |
|---|---|---|---|---|---|
| 1 | 1 | 8.858 | 3.261 | 0.336 | 0.348 |
| 3 | 1 | 5.798 | 2.998 | 0.389 | 0.276 |
| 5 | 1 | 4.026 | 2.451 | 0.522 | 0.315 |
| 6 | 1 | 3.032 | 2.199 | 0.514 | 0.407 |

## Claims

1. Process for the preparation of a dialkyl carbonate and an alkanediol, comprising:
(i) reacting an alkylene carbonate and an alkanol to obtain a product mixture containing unconverted alkylene carbonate, unconverted alkanol, dialkyl carbonate and alkanediol;
(ii) contacting the product mixture obtained in step (i) with an extraction solvent and separating the extraction solvent phase comprising unconverted alkanol and alkanediol from the organic carbonate phase comprising unconverted alkylene carbonate and dialkyl carbonate, wherein the extraction solvent comprises an alkanol containing 2 or more hydroxyl groups;
(iii) recovering the dialkyl carbonate;
(iv) separating the extraction solvent phase obtained in step (ii) into a top stream comprising unconverted alkanol and a bottom stream comprising extraction solvent and alkanediol; and
(v) recycling extraction solvent from the bottom stream obtained in step (iv) to step (ii).

2. Process according to claim 1, wherein in step (i) the alkylene carbonate is a C₂-C₆ alkylene carbonate, suitably a C₂-C₄ alkylene carbonate, more suitably a C₂-C₃ alkylene carbonate, and the alkanol is a C₁-C₄ alkanol, suitably a C₁-C₃ alkanol, more suitably a C₂-C₃ alkanol.

3. Process according to claim 2, wherein in step (i) the alkylene carbonate is ethylene carbonate or propylene carbonate, the alkanol is ethanol and the dialkyl carbonate is diethyl carbonate.

4. Process according to any one of claims 1 to 3, wherein the extraction solvent comprises an alkanol containing of from 2 to 5 hydroxyl groups, preferably of from 2 to 4 hydroxyl groups, more preferably of from 2 to 3 hydroxyl groups, most preferably 3 hydroxyl groups.

5. Process according to claim 4, wherein the extraction solvent comprises monoethylene glycol, monopropylene glycol, glycerol, erythritol, xylitol or sorbitol, preferably monoethylene glycol, monopropylene glycol or glycerol, more preferably monoethylene glycol or glycerol, most preferably glycerol.

6. Process for making a diaryl carbonate, comprising contacting, in the presence of a transesterification catalyst, an aryl alcohol with dialkyl carbonate recovered in step (iii) of the process of any one of claims 1 to 5.

7. Process for making a diaryl carbonate, comprising the preparation of a dialkyl carbonate and an alkanediol in accordance with the process of any one of claims 1 to 5, and then contacting, in the presence of a transesterification catalyst, an aryl alcohol with dialkyl carbonate recovered in step (iii) of the process of any one of claims 1 to 5.
